# EUROPÄISCHE PATENTSCHRIFT

(11) **EP 1 208 796 B1**
(45) Veröffentlichungstag und Bekanntmachung des Hinweises auf die Patenterteilung: **05.03.2003**
(21) Anmeldenummer: 00125227.9
(22) Anmeldetag: 22.11.2000
(51) Int. Cl.: A61B 6/00

(54) **Verfahren zur Bestimmung der Lungenfüllung**
Method for determining lung volume
Procédé de détermination du volume des poumons

(43) Veröffentlichungstag der Anmeldung: 29.05.2002
(73) Patentinhaber: BrainLAB AG, 85551 Kirchheim/Heimstetten (DE)
(72) Erfinder: Erbel, Stephan, 81677 München (DE)
(74) Vertreter: Schwabe - Sandmair - Marx

(56) Entgegenhaltungen:
- DE-A- 19 829 224
- US-A- 5 287 276
- US-A- 5 538 494
- US-A- 5 588 439
- KUBO H D ET AL: "RESPIRATION GATED RADIOTHERAPY TREATMENT: A TECHNICAL STUDY" PHYSICS IN MEDICINE AND BIOLOGY,GB,TAYLOR AND FRANCIS LTD. LONDON, Bd. 41, Nr. 1, 1996, Seiten 83-91, XP000548771 ISSN: 0031-9155

## Beschreibung

Die Erfindung betrifft ein Verfahren zur Bestimmung der aktuellen Lungenfüllung gemäß dem Anspruch 1 und ein Verfahren zur Bestrahlungsunterstützung bei atmungsbedingten Verschiebungen eines Bestrahlungsziels gemäß dem Anspruch 10.

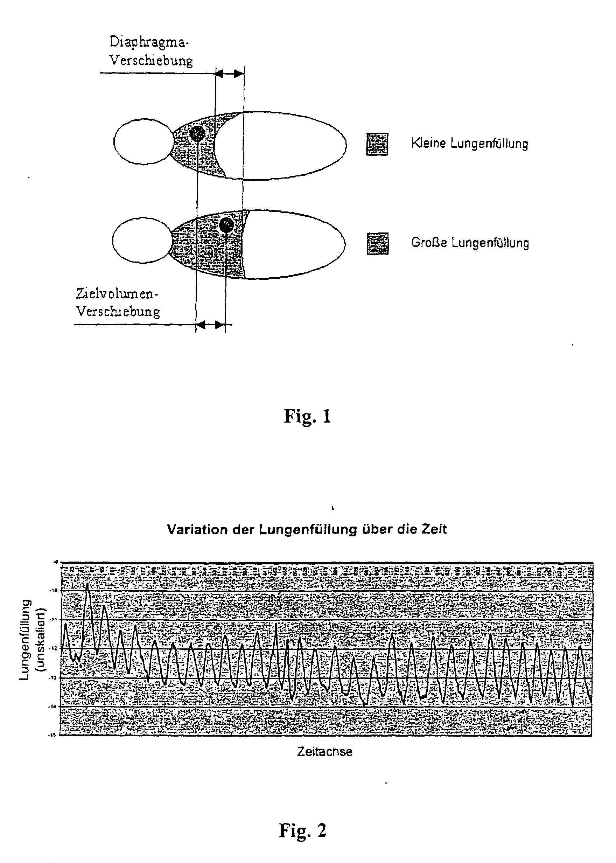

Das Bestrahlen von Tumoren in Bereich der Lunge, Leber und Nieren, wird maßgeblich dadurch erschwert, dass diese Bereiche eine atembedingte Verschiebung erfahren. Um diese Verschiebung zu erfassen beziehungsweise zu messen, gibt es derzeit nur unzulängliche Systeme, In der heutigen klinischen Anwendung werden die Patienten im CT oder in einem Durchleuchtungsbild "kartografiert", d. h. ein Planungsdatensatz wird erstellt, und ein Zielvolumen wird eingezeichnet und gegenüber einem Patientenkoordinatensystem lokalisiert (zum Beispiel mit Hautmarkierungen). Das Problem dabei ist jedoch, dass die Position der angesprochenen Zielvolumina von der Luftmenge beeinflusst wird, die der Patient während der Aufnahme des CTs in seinen Lungen hat. Die während der Planung festgestellte Position des Zielvolumens gilt also nur für genau eine Lungenfüllung. In der Figur 1 ist die Verschiebung des Zielvolumens bei Variieren der Lungenfüllung beim frei atmenden Patienten dargestellt.

Die Lungenfüllung des Patienten ist die Größe, welche die Position des Zielvolumens im Körper des Patienten beeinflusst. Verfolgt man die Lungenfüllung des Patienten über die Zeit, so stellt man fest, dass diese sich mit jedem Atemzug verändert (dies sind die etwa 0,5 Liter Luft, die der Patient ein- und ausatmet). Verfolgt man die Lungenfüllung jedoch über längere Zeit, so stellt man fest, dass sich das Maximum, das Minimum und die Mittellage der Atmung über die Zeit hin ebenfalls verändern, wie dies in Figur 2 dargestellt ist, welche die Variation der Lungenfüllung über die Zeit ersichtlich macht.

Es ist demnach für einen beliebig gewählten Zeitpunkt (bzw. kurzen Zeitraum) in der Zukunft nicht vorherzusagen wo sich das Maximum, das Minimum und die Mittellage der Lungenfüllung eines Patienten befinden werden.

Es besteht das Problem der mangelnden Übertragbarkeit eines 3D-Planungsdatensatzes auf den Zustand des Patienten zum Zeitpunkt der Bestrahlung. Wurde der Planungsdatensatz des Patienten bei angehaltener Atmung aufgenommen (im Normalfall ist dies das Planungs-CT), so stellt dieser Datensatz den Patienten und die Position des Zielvolumens bei einer bestimmten Lungenfüllung dar. Um diese Daten auf den Zustand des Patienten zum Zeitpunkt der Bestrahlung übertragen zu können müssten die Lungenfüllungen zu beiden Zeitpunkten genau übereinstimmen. Kann der Patient während der Bestrahlung frei atmen, so ist dies zu höchstens zwei Zeitpunkten pro Atemzug der Fall. Da Patienten, die für das Planungs-CT ihre Atmung für etwa 10 bis 20 Sekunden anhalten müssen, dazu neigen, davor etwas stärker oder schwächer als bei normaler Atmung ein- oder auszuatmen, kann es auch vorkommen, dass der Patient während des Bestrahlungstermins zu keinem Zeitpunkt die gleiche Lungenfüllung wie auf dem Referenz-(Planungs-)Datensatz hat. In diesem Fall ist die Position des Zielvolumens zu keinem Zeitpunkt bekannt, eine punktgenaue Bestrahlung ist somit unmöglich.

Es wäre deshalb bei vielen medizinischen Anwendungen vorteilhaft, die Atmung des Patienten genau erfassen zu können, zum Beispiel um die Position von Organen oder Tumoren verfolgen zu können, welche durch die Atmung beeinflusst werden. Zu diesem Zweck wurden bereits Verfahren entwickelt, welche die Veränderung äußerer Parameter des Patienten erfassen, und es so erlauben die Atmung des Patienten zu erfassen. Auch durch Spirometrie lässt sich die Atmung des Patienten verfolgen. Problematisch bei diesen Lösungen ist jedoch, dass die Atmung nur verfolgt werden kann, solange der Patient mit dem System in Kontakt bleibt. Ist es jedoch notwendig die Atmung zu vergleichen, die der Patient zu zwei etwas weiter auseinanderliegenden Zeitpunkten hatte, so müssten die obengenannten Systeme permanent mit dem Patienten in Kontakt bleiben. Da dies bei vielen medizinischen Anwendungen nicht möglich oder unerwünscht ist, ist dieser ununterbrochene Kontakt in der Regel nicht gegeben. Bei allen bisher bekannten Verfahren stellt sich dadurch das Problem, dass es nicht möglich ist den Nullpunkt oder einen anderen absoluten Referenzpunkt mit zufriedenstellender Genauigkeit wiederzufinden. Messwerte, zwischen deren Akquisition die Messung unterbrochen wurde, sind somit nicht mehr direkt vergleichbar. Die Ursache für die nicht zufriedenstellende Genauigkeit ist zum Beispiel das Verrutschen von Hautfettschichten und darauf aufgebrachter Referenzmarkierungen, oder bei der Spirometrie die mangelnde Reproduzierbarkeit der maximalen und minimalen Lungenfüllung.

Es ist die Aufgabe der vorliegenden Erfindung, ein Verfahren zur Bestimmung der aktuellen Lungenfüllung und ein Verfahren zur Bestrahlungsunterstützung bei atmungsbedingten Verschiebungen eines Bestrahlungsziels bereitzustellen, welche die oben aufgezeigten Probleme lösen. Insbesondere soll die Bestrahlung von atmungsbedingt verschiebbaren Bestrahlungszielen optimiert werden.

Gelöst wird diese Aufgabe erfindungsgemäß zunächst durch ein Verfahren zur Bestimmung der Lungenfüllung, bei dem die Bewegung einer sich mit der Atmung bewegenden anatomischen Struktur bzw. eines oder mehrerer Punkte auf der sich bewegenden anatomischen Struktur, deren Bewegungsbahn mit dem Lungenfüllung hoch korreliert ist, gegenüber dem Ort mindestens einer atmungsortsfesten anatomischen Struktur erfasst wird, und bei dem dann jedem Abstand zwischen den Strukturen ein bestimmter Lungenfüllungswert zugeordnet wird.

Die Erfindung löst also die oben genannten Probleme dadurch, dass ein Parameter verwendet wird, welcher nicht durch das Verrutschen externer Referenzen verfälscht wird, die Atmung des Patienten eindeutig beschreibt und durch eine Messung mit vernünftigem Aufwand erfasst werden kann. Einen solchen Parameter liefert der oben beschriebene Abstand, woraus sich die Möglichkeit ergibt, die Atmung zu jedem Zeitpunkt genau zu verfolgen, d. h. die Lungenfüllung jederzeit und auch bei zeitlich auseinanderliegenden Erfassungen positiv zu bestimmen. Auf der Basis dieser Kenntnis können dann alle weiteren Erfassungen und Behandlungen umso genauer und in optimierter Weise durchgeführt werden.

Bei einer Ausführungsform der Erfindung wird die Erfassung durch Erstellung von Durchleuchtungsbildem, insbesondere Röntgenbildern durchgeführt, wobei vorzugsweise mindestens zwei Aufnahmen erstellt und die restlichen Werte für die Lungenfüllung interpoliert werden.

Ferner kann die Erfassung durch Erstellung von Bildern aus Schichtbildaufnahmeverfahren, zum Beispiel CT-, MR-, PET-Verfahren, durchgeführt werden, wobei vorzugsweise mindestens zwei Aufnahmen erstellt und die restlichen Werte für die Lungenfüllung interpoliert werden. Vorteilhafterweise werden dabei als Aufnahmen digital rekonstruierte Röntgenbilder verwendet, die aus einem Datensatz eines Schichtbildaufnahmeverfahrens erhalten werden.

Besonders vorteilhaft ist die Verwendung solcher Parameter, die zusätzlich zu den drei obigen Anforderungen (kein Verrutschen, eindeutige Beschreibung der Atmung des Patienten und Messung mit vernünftigem Aufwand) sowohl bei einer Durchleuchtung als auch in dreidimensionalen Planungsdatensätzen erfasst werden können. Erfindungsgemäß ist deshalb vorteilhafterweise die sich mit der Atmung bewegende anatomische Struktur eine bei einer Durchleuchtung, insbesondere auf einer Röntgenaufnahme erkennbare Struktur, bevorzugt das Lungendiaphragma bzw. ein Punkt auf dem Lungendiaphragma (Zwerchfell).

Es wird also ein charakteristischer Punkt einer Struktur der menschlichen Anatomie, welcher durch die Atmung mitbewegt wird und sowohl im Röntgenbild als auch in dreidimensionalen Planungssätzen sichtbar ist, betrachtet, zum Beispiel das Lungendiaphragma. Wird der Abstand zwischen dieser Referenz zu der atmungsortsfesten (also durch die Atmung nicht mitgeführten) anatomischen Struktur, zum Beispiel ein Wirbelkörper oder ein Wirbelkörperteil, insbesondere eine Wirbelkörperkante, ermittelt und verfolgt, so erhält man einen Wert, welcher zur Lungenfüllung des Patienten sehr hoch korreliert ist.

Alternativ ist es ebenfalls möglich den scheinbaren Abstand zwischen den beiden Referenzen in einem zweidimensionalen Bild zu vermessen. Damit die Messwerte aus verschiedenen zweidimensionalen Abbildungen direkt verglichen werden können, ist es jedoch notwendig, dass die Projektionsparameter beider Abbildungen identisch sind (Ansichtswinkel und Entfernung entsprechen einander bei digital rekonstruierten Röntgenbildern und echten Röntgenaufnahmen). Die Erfindung fasst demgemäss auch ins Auge, dass eine erste Erfassung durch Erstellung von Durchleuchtungsbildern, insbesondere Röntgenbildern durchgeführt wird, und eine zweite Erfassung durch Erstellung von entsprechenden Bildern aus Schichtbildaufnahmeverfahren, zum Beispiel CT-, MR-, PET-Verfahren, durchgeführt wird, wobei immer vorzugsweise mindestens zwei Aufnahmen erstellt und die restlichen Werte für die Lungenfüllung interpoliert werden, und wobei ein Vergleich bzw. Abgleich der bestimmten Lungenfüllungswerte erfolgt.

Ein weiterer Aspekt der Erfindung betrifft ein Verfahren zur Unterstützung einer Bestrahlung bei atmungsbedingten Verschiebungen eines Bestrahlungsziels, gemäß Anspruch 10.

Mit anderen Worten umfasst dieses Verfahren also das Feststellen sowohl der Lungenfüllung, die der Patient während der Aufnahme eines oder mehrerer Planungsdatensätze hatte, als auch der Lungenfüllung, die der Patient während der Bestrahlung hat. Durch eine Verknüpfung der entsprechenden Informationen lässt sich dann mit der bekannten Lungenfüllung als Bindeglied mittels der Daten aus dem Planungsdatensatz (diese enthalten die Position des Bestrahlungsziels) und der Daten aus der aktuellen Durchleuchtung (diese enthalten meist nicht die Position des Behandlungsziels, insbesondere bei Röntgenaufnahmen, mit denen zum Beispiel ein Tumor oft nicht ausreichend sichtbar gemacht werden kann, aber sie enthalten die aktuelle Position von positionsverfolgbaren Trackingeinrichtungen, zum Beispiel Markierungen) auch die Position des Bestrahlungsziels bzw. dessen Bewegung ermitteln. Beide Datensätze enthalten nämlich den Abstand zwischen beweglicher und ortsfester Struktur, der auf die Lungenfüllung schließen lässt. Die Position des Zielvolumens wird insbesondere bei mehreren Lungenfüllungen bestimmt, so dass im Folgenden aus der Lungenfüllung die Position des Zielvolumens errechnet werden kann.

Bei einer Ausführungsvariante wird die aktuelle Bestrahlungszielposition auf der Basis des Lungenfüllungswerts dadurch erfasst, dass
- der Positionsverlauf eines Bestrahlungsziels bei der Erfassung der Bewegung der sich bewegenden Struktur mit erfasst wird und jedem Abstandswert, d. h. jedem Lungenfüllungswert ein Positionswert des Bestrahlungsziels zugeordnet wird,
- vor der Bestrahlung mindestens eine, vorzugsweise mindestens zwei Durchleuchtungen erfolgen, bei denen die Bewegung der sich bewegenden anatomischen Struktur gegenüber der Position einer positionsgebenden Trackingeinrichtung, insbesondere Markierung am Patienten erfasst wird und dann jedem Abstand zwischen der Struktur und den Markierungen ein bestimmter Lungenfüllungswert zugeordnet wird,
- aus den Zuordnungen von Bestrahlungszielposition zu Lungenfüllungswert und Lungenfüllungswert zur Positionsinformation der Trackingeinrichtung, insbesondere Markierungsposition eine Abhängigkeit der aktuellen Bestrahlungszielposition von der Positionsinformation der Trackingeinrichtung, insbesondere der Markierungsposition ermittelt wird.

Die Zuordnung der Lungenfüllwerte zu dem Abstand der sich bewegenden Struktur und der atmungsortsfesten Struktur kann mittels digital aus einem Schichtbildaufnahmedatensatz rekonstruierter Röntgenbilder (DRRs) ermittelt werden, welche in Winkellage und Aufnahmeentfernung den Durchleuchtungen vor oder bei der Bestrahlung entsprechen.

Die aktuelle Bestrahlungszielposition kann in Echtzeit oder zeitversetzt auf der Basis des Lungenfüllungswerts erfasst werden.

Die Positionsinformation der Trackingeinrichtung kann erhalten werden durch Auswertung der Daten mindestens einer der folgenden Einrichtungen bzw. Verfahren, die atembeeinflusste Parameter liefern:
- Markierungen, insbesondere reflektierende Markeranordnungen, vorzugsweise für Infrarotlicht, die mittels eines computergestützten Trackingsystems positionell im Raum erfasst und verfolgt werden können,
- Konturüberwachung des Patienten durch Videokameras, zum Beispiel durch Interferenzmuster oder mittels polarisiertem Licht,
- Dehnungsmessbänder,
- Spirometrie,
- Elektromyographie,
- mechanisches Abtasten eines oder mehrerer Punkte auf der Patientenoberfläche, insbesondere mittels einer Koordinatenmessmaschine.

Die Kenntnis der aktuellen Bestrahlungszielposition kann zur Steuerung des Ein- und Ausschalten des Therapiestrahls verwendet und insbesondere dazu genutzt werden, den Therapiestrahl nur dann einzuschalten, wenn das Bestrahlungsziel innerhalb eines wählbaren Toleranzbereiches um das Bestrahlungsziel bzw. die Bestrahlungsachse eines Therapiegerätes liegt. Ferner oder zusätzlich kann diese Kenntnis der aktuellen Bestrahlungszielposition, also auch des kopfnähesten und fußnähesten Punktes, dazu genutzt werden, einen der Endpunkte von deren Bewegungsbahn, relativ zum Zielpunkt oder der Zielachse eines Therapiegerätes, so zu positionieren, dass bei atemgesteuerter Bestrahlung eine optimal hohe anteilige Bestrahlungszeit erreicht wird. Nach dem Ausatmen bleibt das Bestrahlungsziel bzw. das Zielvolumen für fast eine Sekunde lang unbewegt. Aus diesem Grund ist es besonders sinnvoll das Zielvolumen zu diesem Zeitpunkt zu bestrahlen, da man eine akzeptable anteilige Bestrahlungszeit bei minimaler residueller Bewegung des Zielvolumens erhält

Außerdem ist es möglich, die Kenntnis der aktuellen Bestrahlungszielposition dazu zu nutzten, eine dynamische Repositionierung während der Bestrahlung vorzunehmen, um mittelfristige Schwankungen der Randwerte, nämlich Minimum und Maximum, der Lungenfüllung des Patienten zu berücksichtigen. Hat der Patient während der Atmung zum Beispiel zunehmend weniger Luft in seiner Lunge, so verschiebt sich die Mittellage des Zielvolumens im Patienten; ebenso verschieben sich die beiden Extrempunkte der momentanen Bewegungsbahn. Dies kann dazu führen, dass das Zielvolumen den Zielpunkt/die Zielachse des Bestrahlungsgerätes nicht mehr durchquert, bzw. dass das Zielvolumen den Zielpunkt/die Zielachse des Bestrahlungsgerätes zu schnell durchquert. Dies kann durch ein entsprechendes Verschieben des Patienten relativ zum Bestrahlungsgerät ausgeglichen werden.

Die Bewegung des Bestrahlungsziels innerhalb des Patienten kann durch ein kontinuierliches, gegenläufiges Bewegen des Patienten auf einer Patientenliege ausgeglichen werden.

Eine Ausführungsform der Erfindung wird im Weiteren erläutert. In den Zeichnungen zeigen:
- Figur 1: die Verschiebung des Zielvolumens bei Variieren der Lungenfüllung beim frei atmenden Patienten;
- Figur 2: die Variation der Lungenfüllung über die Zeit;
- Figur 3: eine schematische Darstellung der Berechnung eines digital rekonstruierten Röntgenbildes (DRR);
- Figur 4: eine Gegenüberstellung eines DRR und eines entsprechenden Röntgenbildes;
- Figur 5: ein Durchleuchtungsbild, in dem das Bestrahlungsziel, und der Abstand zwischen dem Lungendiaphragma und einem charakteristischen, atmungsortsfesten Skelettpunkt bezeichnet sind;
- Figur 6: Lungenfüllung und Tumorposition in zwei verschiedenen Atmungszuständen;
- Figur 7: die Position des Bestrahlungsziels (Zielvolumen), in einem Diagramm aufgetragen über der Position des Lungendiaphragmas (Lungenfüllung);
- Figur 8: die Position von Markern, in einem Diagramm aufgetragen über der Position des Lungendiaphragmas (Lungenfüllung); und
- Figur 9: den diagrammatischen Rückschluss von der Position der Marker auf die Position des Bestrahlungsziels (Zielvolumen).

Die Ausführungsform der Erfindung wird im folgenden mittels einer chronologischen Darstellung der Vorgehensweise erläutert.

Anfangs erfolgt eine Aufnahme der Planungsdatensätze. Im Gegensatz zur konventionellen Vorgehensweise wird der Patient nicht nur einmal gescannt, sonder mindestens zweimal. Als bildgebende Verfahren kommen dabei alle Schichtbildverfahren in Frage (MR, CT, PET, ...). Notwendig ist hierbei, dass die Aufnahmen bei angehaltener Atmung angefertigt werden, und dass sich die Lungenfüllung bei den verschiedenen Aufnahmen unterscheidet (vorzugsweise ein Scan nach dem Einatmen, ein Scan nach dem Ausatmen). In beiden Datensätzen wird auf herkömmliche Weise das Zielgebiet definiert.

Bei jedem Bestrahlungstermin werden dem Patienten ein oder mehrere Marker auf den Thorax geklebt, die sich mit der Atmung des Patienten verschieben. Die Position dieser Marker wird in Echtzeit über ein computer- und kameraunterstütztes Trackingsystem verfolgt. Über einen Atemzyklus verteilt werden nun vom Patienten mindestens zwei Röntgenaufnahmen gemacht (zum Beispiel mit Röntgenquellen an der Raumdecke; das Bild wird von einem digitalen Detektor unter dem Patienten aufgenommen). Der Ansichtswinkel aus dem die Röntgenbilder aufgenommen wurden, muss dabei bekannt sein; die Position der Marker auf dem Thorax des Patienten zum Zeitpunkt der Bildaufnahme wird abgespeichert.

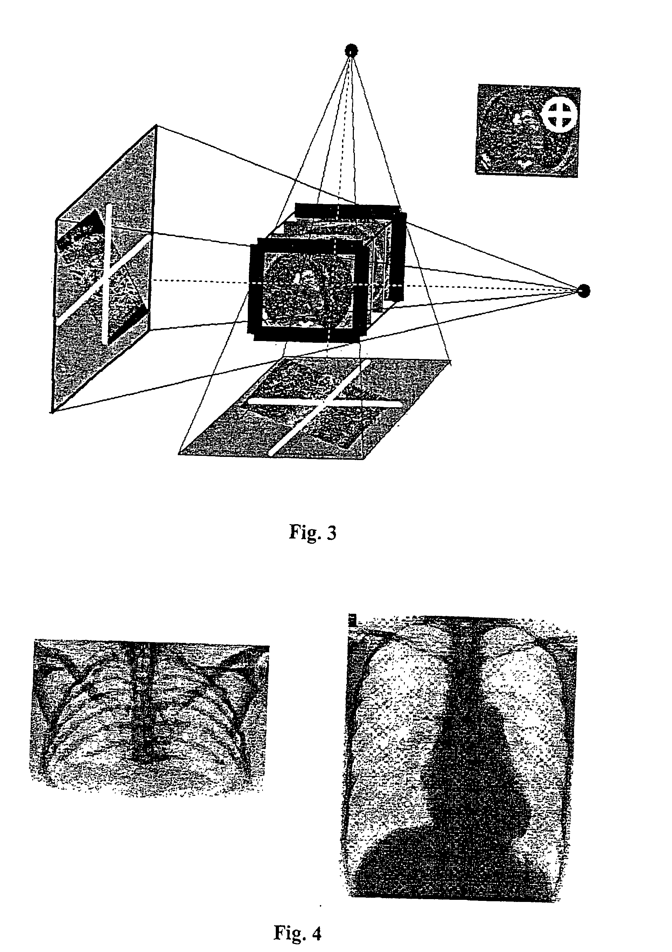

Durch entsprechende geometrische Berechnungen lässt sich aus den 3D-Planungsdatensätzen jeweils ein "Digital Rekonstruiertes Röntgenbild" (DRR) errechnen, das zu dem Ansichtswinkel passt, aus dem die Röntgenaufnahmen gemacht wurden. In der Figur 3 ist schematisch dargestellt, wie ein solches Bild errechnet wird, das auch die Positionsinformationen des Bestrahlungsziels enthält. Eine Gegenüberstellung von DRR (links) und entsprechender Röntgenaufnahme des Thorax ist in Figur 4 zu sehen.

Der Bildbereich für die Röntgenaufnahmen und die DRRs wird so gewählt, dass auf dem Bild das Lungendiaphragma zu erkennen ist. (Das Lungendiaphragma ist sozusagen die Grenze zwischen der Lunge und der darunter liegenden Leber.) Aufgrund des hohen Dichteunterschiedes zwischen Leber und Lunge ist diese Trennlinie sowohl in den DRRs als auch in den Röntgenbildern zu erkennen. Die Position des Lungendiaphragmas im Brustkorb des Patienten definiert die Lungenfüllung des Patienten zu dem jeweiligen Zeitpunkt.

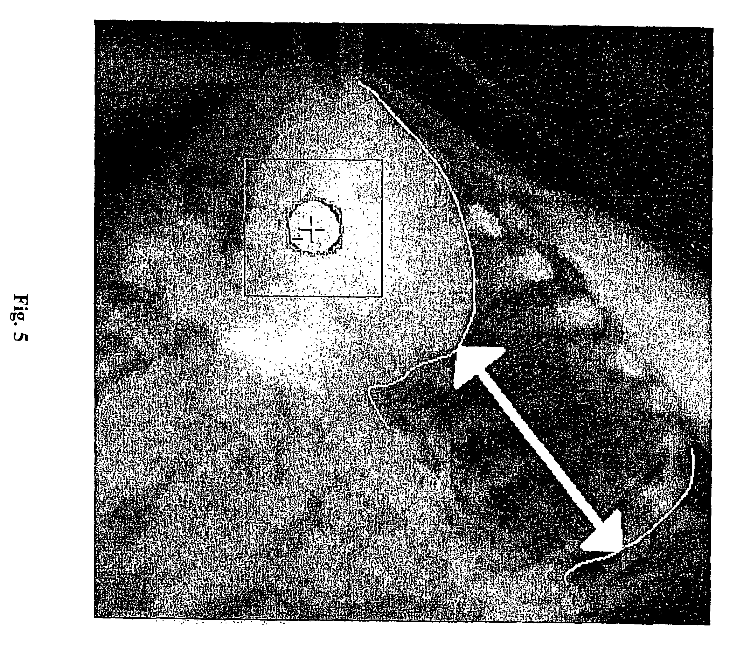

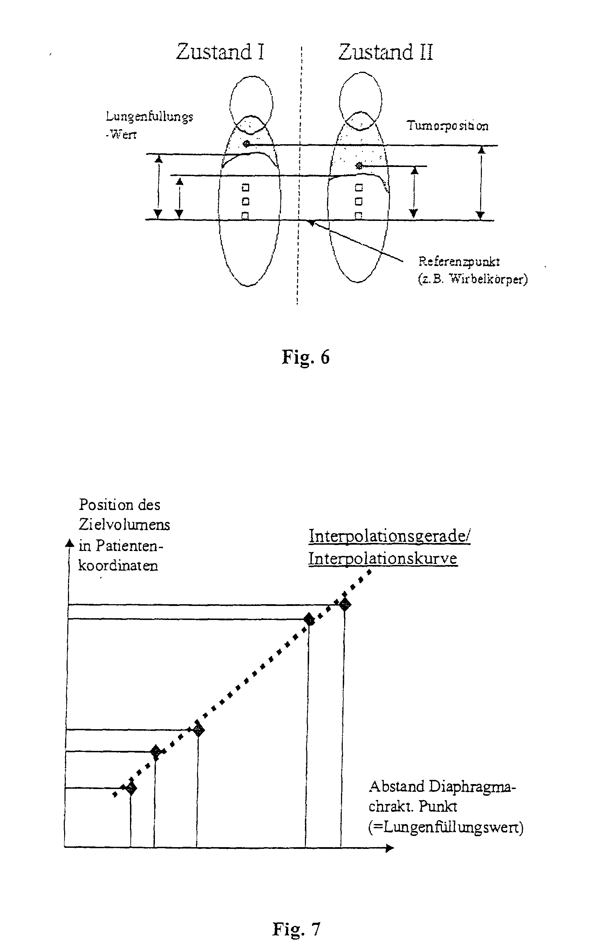

In den vorliegenden Bildern lässt sich, wie beispielhaft in Figur 5 gezeigt, demnach von Hand oder automatisiert das Diaphragma einzeichnen. Wird in jedem Bild der Abstand zwischen einem charakteristischen Punkt der sich nicht mit der Atmung verschiebt (dies kann zum Beispiel eine Kontur eines Wirbelkörpers oder eine Rippe sein) und eines Punktes auf dem Diaphragma vermessen, so lässt sich dem entsprechenden Bild ein Wert zuordnen, der die Lungenfüllung des Patienten eindeutig beschreibt. Der auf diese Weise gemessene Wert soll im folgenden als "Lungenfüllungswert" bezeichnet werden. Die Figur 6 zeigt schematisch den Abstand zwischen dem Diaphragma und einem charakteristischen Punkt des Skeletts bei zwei verschiedenen Atmungszuständen auf (Zustände I und II).

Die so erzeugten Daten werden nun paarweise in Relation zueinander gesetzt und man erhält eine Beziehung zwischen der Tumorposition und dem Lungenfüllungswert (Diaphragmaposition). Hier ist anzumerken, dass die zur besseren Verständlichkeit vereinfachten Abbildungen die Vorgehensweise lediglich für eine Raumkoordinate (Kopf-Fuß-Richtung) zeigen. Die Position des Tumors muss jedoch in allen drei Raumkoordinaten bestimmt werden, und die Korrelationen für alle Achsen bestimmt werden.

In den Planungsdatensätzen ist sowohl die Position des Zielvolumens, als auch der zu dieser Position des Zielvolumens passende Lungenfüllungswert abzulesen. Es lässt sich demnach eine Interpolationsgerade durch diese Wertepaare legen, welche den Zusammenhang zwischen beiden Größen beschreibt. Dies ist in der Figur 7 gezeigt, in welcher die Position des Zielvolumens aufgetragen ist über der Position des Diaphragmas (Lungenfüllungswert); die Interpolationskurve ist eingezeichnet, und sie kann gut linear angenähert werden.

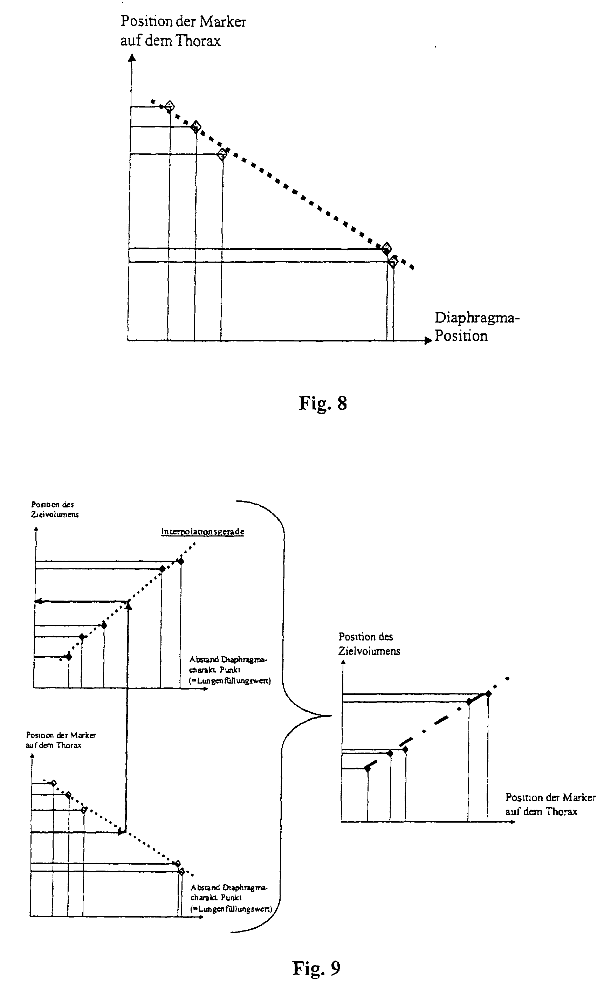

Analog ist bei den Röntgenbildern jeweils die Position der (IR-reflektierenden) Marker und der dazu passende Lungenfüllungswert bekannt. Durch diese Wertepaare lässt sich nun ebenfalls eine Interpolationsgerade legen, die den Zusammenhang zwischen diesen beiden Größen beschreibt. Die Figur 8, welche Position der Marker auf dem Thorax aufgetragen über die Lungenfüllung zeigt, enthält auch diese zweite Interpolationslinie.

Mathematisch lassen sich diese beiden Interpolationsfunktionen so verknüpfen, dass sie anhand der Position der reflektierenden Marker auf die Position des Zielvolumens schließen lassen. Graphisch ist dieser Rückschluss von der Position der Marker auf die Position des Zielvolumens in der Figur 9 dargestellt. Hier sind die beiden Diagramme aus den Figuren 7 und 8 auf der linken Seite übereinander gestellt. Die fette Linie zeigt die Vorgehensweise an. Zunächst wird im unteren Diagramm aus der Markerposition der Lungenfüllungswert ermittelt. Mit dieser Information geht man in das obere Diagramm und erhält zum ermittelten Lungenfiillungswert die Position des Zielvolumens.

Dies bedeutet nun aber, dass, wenn die Position der Marker in Echtzeit verfolgt wird, sich auch die Position des Zielvolumens in Echtzeit errechnen lässt. Anhand der in Echtzeit bekannten Position des Zielvolumens lässt sich nun die atemgesteuerte Bestrahlung des Patienten mit sehr hoher Genauigkeit durchführen.

Gegenüber der direkten Erfassung der Position der aufgeklebten Marker hat das erfindungsgemäße Verfahren einen großen Vorteil, der in der Genauigkeit und Zuverlässigkeit liegt. Dies liegt daran, dass die Positionstreue der Marker durch das Verrutschen auf den Fettschichten nicht optimal ausreichend ist, wenn der Patient zwischen der Erfassung des Planungsdatensatzes und der eigentlichen Bestrahlung bewegt wird. Dies wird aber immer der Fall sein, wenn er vom CT in den Bestrahlungsraum gebracht werden muss.

Marker können nur solange zuverlässig in Referenz gesetzt werden, wie ein Patient ruhig liegen bleibt. Sobald er aufsteht bzw. die Marker abgenommen werden ist die Zuordnung ungenau. Ein direktes Zuordnen von Tumorposition und Markerposition ist deshalb nicht optimal. Eine erfindungsgemäße Zuordnung Tumor-Diaphragma im CT und eine noch "warme" Zuordnung Diaphragmapositions-Marker am Linearbeschleuniger, bei der das Verfahren zur Bestimmung der Lungenfüllung gemäß der vorliegenden Erfindung eingesetzt wird, löst diese Probleme.

## Patentansprüche

1. Verfahren zur Bestimmung der Lungenfüllung, bei dem die Bewegung einer sich mit der Atmung bewegenden anatomischen Struktur bzw. eines oder mehrerer Punkte auf der sich bewegenden anatomischen Struktur, deren Bewegungsbahn mit dem Lungenfüllung hoch korreliert ist, gegenüber dem Ort mindestens einer atmungsortsfesten anatomischen Struktur erfasst wird, und bei dem dann jedem Abstand zwischen den Strukturen ein bestimmter Lungenfüllungswert zugeordnet wird.

2. Verfahren nach Anspruch 1, bei dem die Erfassung durch Erstellung von Durchleuch-Röntgenbildern durchgeführt wird.

3. Verfahren nach Anspruch 2, bei dem die Erfassung durch Erstellung von Röntgenbildem durchgeführt wird.

4. Verfahren nach Anspruch 1 oder 2, bei dem die Erfassung durch Erstellung von Bildern aus Schichtbildaufnahmeverfahren durchgeführt wird.

5. Verfahren nach einem der Ansprüche 2 bis 4, bei dem mindestens zwei Aufnahmen erstellt und die restlichen Werte für die Lungenfüllung interpoliert werden.

6. Verfahren nach Anspruch 4 oder 5, bei dem als Aufnahmen digital rekonstruierte Röntgenbilder verwendet werden, die aus einem Datensatz eines Schichtbildaufhahmeverfahrens erhalten werden.

7. Verfahren nach einem der Ansprüche 1 bis 6, bei dem die sich mit der Atmung bewegende anatomische Struktur das Lungendiaphragma bzw. ein Punkt auf dem Lungendiaphragma ist.

8. Verfahren nach einem der Ansprüche 1 bis 7, bei dem die atmungsortsfeste anatomische Struktur ein Wirbelkörper oder ein Wirbelkörperteil ist.

9. Verfahren nach einem der Ansprüche 1 bis 8, bei dem eine erste Erfassung durch Erstellung von Durchleuchtungsbildern durchgeführt wird, und bei dem eine zweite Erfassung durch Erstellung von entsprechenden Bildern aus Schichtbildaufnahmeverfahren durchgeführt wird, wobei immer mindestens zwei Aufnahmen erstellt und die restlichen Werte für die Lungenfüllung interpoliert werden, und bei dem ein Vergleich bzw. Abgleich der bestimmten Lungenfüllungswerte erfolgt.

10. Verfahren zur Unterstützung einer Bestrahlung bei atmungsbedingten Verschiebungen eines Bestrahlungsziels, bei dem
- mittels eines Verfahrens nach einem der Ansprüche 1 bis 7 die Zuordnung der Lungenfüllwerte zu dem Abstand der sich bewegenden Struktur, welche bei einer Durchleuchtung, insbesondere auf einer Röntgenaufnahme erkennbar ist, und der atmungsortsfesten Struktur ermittelt wird,
- die aktuelle Bestrahlungszielposition auf der Basis des Lungenfüllungswerts erfasst wird.

11. Verfahren nach Anspruch 10, bei dem die aktuelle Bestrahlungszielposition auf der Basis des Lungenfüllungswerts dadurch erfasst wird, dass
- der Positionsverlauf eines Bestrahlungsziels bei der Erfassung der Bewegung der sich bewegenden Struktur mit erfasst wird und jedem Abstandswert, d. h. jedem Lungenfüllungswert ein Positionswert des Bestrahlungsziels zugeordnet wird,
- vor der Bestrahlung mindestens eine Durchleuchtung erfolgt, bei der die Bewegung der sich bewegenden anatomischen Struktur gegenüber der Position einer positionsgebenden Trackingeinrichtung am Patienten erfasst wird, und bei dem dann jedem Abstand zwischen der Struktur und der Trackingeinrichtung ein bestimmter Lungenfüllungswert zugeordnet wird,
- aus den Zuordnungen von Bestrahlungszielposition zu Lungenfüllungswert und Lungenfüllungswert zur Positionsinformation der Trackingeinrichtung eine Abhängigkeit der aktuellen Bestrahlungszielposition von der Positionsinformation der Trackingeinrichtung ermittelt wird.

12. Verfahren nach Anspruch 10 oder 11, bei dem die Zuordnung der Lungenfüllwerte zu dem Abstand der sich bewegenden Struktur und der atmungsortsfesten Struktur mittels digital aus einem Schichtbildaufnahmedatensatz rekonstruierter Röntgenbilder (DRRs) ermittelt wird, welche in Winkellage und Aufnahmeentfernung den Durchleuchtungen vor der Bestrahlung entsprechen.

13. Verfahren nach einem der Ansprüche 10 bis 12, bei dem die aktuelle Bestrahlungszielposition in Echtzeit oder zeitversetzt auf der Basis des Lungenfüllungswerts erfasst wird.

14. Verfahren nach einem der Ansprüche 10 bis 13, bei dem die Positionsinformation der Trackingeinrichtung erhalten wird durch Auswertung der Daten mindestens einer der folgenden Einrichtungen bzw. Verfahren, die atembeeinflusste Parameter liefern:
- Markierungen, insbesondere reflektierende Markeranordnungen, vorzugsweise für Infrarotlicht, die mittels eines computergestützten Trackingsystems positionell im Raum erfasst und verfolgt werden können,
- Konturüberwachung des Patienten durch Videokameras, zum Beispiel durch Interferenzmuster oder mittels polarisiertem Licht,
- Dehnungsmessbänder,
- Spirometrie,
- Elektromyographie,
- mechanisches Abtasten eines oder mehrerer Punkte auf der Patientenoberfläche, insbesondere mittels einer Koordinatenmessmaschine.

15. Verfahren nach einem der Ansprüche 10 bis 14, bei dem die Kenntnis der aktuellen Bestrahlungszielposition zur Steuerung des Ein- und Ausschalten des Therapiestrahls verwendet und insbesondere dazu genutzt wird, den Therapiestrahl nur dann einzuschalten, wenn das Bestrahlungsziel innerhalb eines wählbaren Toleranzbereiches um das Bestrahlungsziel bzw. die Bestrahlungsachse eines Therapiegerätes liegt.

16. Verfahren nach einem der Ansprüche 10 bis 15, bei dem die Kenntnis der aktuellen Bestrahlungszielposition, also auch des kopfnähesten und fußnähesten Punktes, dazu genutzt wird, einen der Endpunkte von deren Bewegungsbahn, relativ zum Zielpunkt oder der Zielachse eines Therapiegerätes, so zu positionieren, dass bei atemgesteuerter Bestrahlung eine optimal hohe anteilige Bestrahlungszeit erreicht wird.

17. Verfahren nach einem der Ansprüche 10 bis 14, bei dem die Kenntnis der aktuellen Bestrahlungszielposition dazu genutzt wird, eine dynamische Repositionierung während der Bestrahlung vorzunehmen, um mittelfristige Schwankungen der Randwerte, nämlich Minimum und Maximum, der Lungenfüllung des Patienten zu berücksichtigen.

18. Verfahren nach Anspruch 17, bei dem die Bewegung des Bestrahlungsziels innerhalb des Patienten durch ein kontinuierliches, gegenläufiges Bewegen des Patienten auf einer Patientenliege ausgeglichen wird.

## Claims

1. A method for determining the filling of a lung, wherein the movement of an anatomical structure which moves with breathing, or of one or more points on a moving anatomical structure whose movement trajectory is highly correlated with lung filling, is detected with respect to the location of at least one anatomical structure which is not spatially affected by breathing, and wherein each distance between the structures is then assigned a particular lung filling value.

2. The method as set forth in claim 1, wherein detection is carried out by producing transillumination images.

3. The method as set forth in claim 2, wherein detection is carried out by producing x-ray images.

4. The method as set forth in claim 1 or 2, wherein detection is carried out by producing images from tomographic imaging methods.

5. The method as set forth in any one of claims 2 to 4, wherein at least two images are produced and the remaining values for lung filling are interpolated.

6. The method as set forth in claim 4 or 5, wherein digitally reconstructed x-ray images, obtained from a data set of a tomographic imaging method, are used as the images.

7. The method as set forth in any one of claims 1 to 6, wherein the anatomical structure which moves with breathing is the pulmonary diaphragm or a point on the pulmonary diaphragm.

8. The method as set forth in any one of claims 1 to 7, wherein the anatomical structure which is not spatially affected by breathing is a vertebral body or a part of a vertebral body.

9. The method as set forth in any one of claims 1 to 8, wherein a first detection is carried out by producing transillumination images, and wherein a second detection is carried out by producing corresponding images from tomographic imaging methods, wherein at least two images are always produced and the remaining values for lung filling are interpolated, and wherein the particular lung filling values are compared and/or aligned.

10. A method for assisting in radiotherapy during movement of the radiation target due to breathing, wherein:
the association of lung filling values with the distance between the moving structure which is identifiable in a transillumination image, in particular an x-ray image, and the structure which is not spatially affected by breathing is determined by means of a method as set forth in any one of claims 1 to 7;
the current position of the radiation target is detected on the basis of the lung filling value.

11. The method as set forth in claim 10, wherein the current position of the target volume is detected on the basis of the lung filling value, by:
- detecting the positional course of a radiation target while detecting the movement of the moving structure, and assigning each distance value, i.e. each lung filling value, a positional value of the radiation target;
- taking at least one transillumination before radiation exposure, in which the movement of the moving anatomical structure with respect to the position of a locational tracking device on the patient is detected, and in which each distance between the structure and the tracking device is then assigned a particular lung filling value;
- determining a dependence of the current position of the radiation target upon the positional information from the tracking device from the associations of the position of the radiation target with the lung filling value, and the lung filling value with the positional information from the tracking.

12. The method as set forth in claim 10 or 11, wherein the assignment of lung filling values to the distance between the moving structure and the structure which is not spatially affected by breathing is determined by means of x-ray images digitally reconstructed from a tomographic imaging data set (DDRs), corresponding in angular position and recording distance to the transilluminations taken before radiation exposure.

13. The method as set forth in any one of claims 10 to 12, wherein the current position of the radiation target is detected on the basis of the lung filling value, in real time or deferred.

14. The method as set forth in any one of claims 10 to 13, wherein the positional information from the tracking device is obtained by evaluating the data of at least one of the following devices and/or methods which provide parameters affected by breathing:
- markings, in particular arrangements of reflecting markers, preferably for infrared light, which may be positionally detected and tracked in space by means of a computer-assisted tracking system;
- monitoring the contours of the patient using video cameras, for instance with an interference pattern or by means of polarised light;
- wire strain gauges;
- spirometry;
- electromyography;
- mechanically scanning one or more points on the surface of the patient, in particular by means of a co-ordinate measuring machine.

15. The method as set forth in any one of claims 10 to 14, wherein the knowledge of the current position of the radiation target is used to control the switching on and off of the therapy beam, and in particular used for only switching the therapy beam on when the radiation target is within a selectable range of tolerance about the radiation target or the axis of radiation of a therapy device.

16. The method as set forth in any one of claims 10 to 15, wherein the knowledge of the current position of the radiation target, and therefore of the points nearest the head and nearest the feet, is used to position one of the end points of its movement trajectory, relative to the target point or the target axis of a therapy device, in such a way that an optimally high proportional radiation exposure time is achieved in breath-controlled radiation exposure.

17. The method as set forth in any one of claims 10 to 14, wherein the knowledge of the current position of the radiation target is used to carry out dynamic repositioning during radiation exposure, to take into account medium-term variations in the boundary values, namely the minimum and maximum, of the patient's lung filling.

18. The method as set forth in claim 17, wherein movement of the radiation target within the patient is compensated for by a continuous, reverse movement of the patient on a patient's table.

## Revendications

1. Procédé pour la détermination du volume des poumons, dans lequel on détecte le déplacement d'une structure anatomique qui se déplace avec la respiration ou d'un ou de plusieurs points sur la structure anatomique en déplacement, dont le parcours de déplacement est fortement corrélé au volume des poumons, par rapport à l'emplacement d'au moins une structure anatomique fixe lors de la respiration et dans lequel une valeur définie du volume des poumons est associée à chaque distance entre les structures.

2. Procédé selon la revendication 1, dans lequel la détection est réalisée par établissement d'images par transparence.

3. Procédé selon la revendication 2, dans lequel la détection est réalisée par établissement d'images par rayons X.

4. Procédé selon la revendication 1 ou 2, dans lequel la détection est réalisée par établissement d'images provenant d'un procédé d'enregistrement d'images tomographiques.

5. Procédé selon l'une des revendications 2 à 4, dans lequel au moins deux enregistrements sont établis et les autres valeurs sont interpolées pour obtenir le volume des poumons.

6. Procédé selon la revendication 4 ou 5, dans lequel on utilise comme enregistrement des images par rayons X reconstruites numériquement qui sont obtenues dans un ensemble de données d'un procédé d'enregistrement d'images tomographiques.

7. Procédé selon l'une des revendications 1 à 6, dans lequel la structure anatomique qui se déplace avec la respiration est le diaphragme des poumons ou un point sur le diaphragme des poumons.

8. Procédé selon l'une des revendications 1 à 7, dans lequel la structure anatomique fixe lors de la respiration est une vertèbre ou une partie d'une vertèbre.

9. Procédé selon l'une des revendications 1 à 8, dans lequel une première détection est réalisée par établissement d'images par transparence, et dans lequel une deuxième détection est réalisée par établissement d'images correspondantes provenant d'un procédé d'enregistrement d'images tomographiques, au moins deux enregistrements étant toujours établis et les autres valeurs du volume des poumons étant interpolées, et dans lequel on effectue une comparaison ou une compensation des valeurs définies du volume des poumons.

10. Procédé d'assistance d'une irradiation lors des déplacements provoqués par la respiration d'une cible d'irradiation, dans lequel:
- l'attribution des valeurs du volume des poumons à la distance de la structure qui se déplace et qui est reconnaissable par transparence, en particulier sur un enregistrement par rayons X, et la structure fixe lors de la respiration est déterminée au moyen d'un procédé selon l'une des revendications 1 à 7, et
- la position effective de la cible d'irradiation est déterminée à partir de la valeur du volume des poumons.

11. Procédé selon la revendication 10, dans lequel la position effective de la cible d'irradiation est détectée à partir de la valeur du volume des poumons,
- en détectant la modification de position d'une cible d'irradiation lors de la détection du déplacement de la structure en déplacement, une valeur de position de la cible d'irradiation étant associée à chaque valeur de distance, c'est-à-dire à chaque valeur du volume des poumons,
- en effectuant avant l'irradiation au moins un éclairage par transparence dans lequel le déplacement de la structure anatomique qui se déplace par rapport à la position d'un dispositif de suivi placé sur le patient et donnant la position est détecté, une valeur définie du volume des poumons étant ensuite associée à chaque distance entre la structure et le dispositif de suivi,
- en déterminant une dépendance entre la position effective de la cible d'irradiation et l'information de position du dispositif de suivi à partir des associations entre la position de la cible d'irradiation et la valeur du volume des poumons et entre la valeur du volume des poumons et les informations de position du dispositif de suivi.

12. Procédé selon la revendication 10 ou 11, dans lequel l'association entre les valeurs de volume des poumons et la distance entre la structure en déplacement et la structure fixe lors de la respiration est déterminée au moyen d'images par rayons X reconstruites numériquement (DRR) à partir d'un ensemble de données d'enregistrement d'images tomographiques dont la position angulaire et la distance d'enregistrement correspondent à celles des éclairages par transparence avant l'irradiation.

13. Procédé selon l'une des revendications 10 à 12, dans lequel la position effective de la cible d'irradiation est déterminée en temps réel ou en différé à partir de la valeur du volume des poumons.

14. Procédé selon l'une des revendications 10 à 13, dans lequel les informations de position du dispositif de suivi sont obtenues par évaluation des données d'au moins l'un des dispositifs ou procédés ci-dessous, qui délivrent des paramètres dépendant de la respiration:
- des repères, particulièrement des agencements de repères réfléchissants, de préférence en lumière infrarouge, dont la position dans l'espace peut être détectée et suivie au moyen d'un système de suivi assisté par ordinateur,
- une surveillance du contour du patient par caméras vidéo, par exemple par motifs d'interférence ou au moyen de lumière polarisée,
- des bandes de mesure d'allongement,
- la spirométrie,
- l'électromyographie,
- la détection mécanique d'un ou de plusieurs points à la surface du patient, en particulier au moyen d'une machine de mesure de coordonnées.

15. Procédé selon l'une des revendications 10 à 14, dans lequel la connaissance de la position effective de la cible d'irradiation est utilisée pour la commande du branchement et du débranchement du rayonnement thérapeutique et est utilisée en particulier pour brancher le rayonnement thérapeutique uniquement lorsque la cible d'irradiation est située à l'intérieur d'une plage sélectionnable de tolérance autour de la cible d'irradiation ou de l'axe d'irradiation d'un appareil thérapeutique.

16. Procédé selon l'une des revendications 10 à 15, dans lequel la connaissance de la position effective de la cible d'irradiation, et donc également du point le plus proche de la tête et du point le plus proche des pieds, est utilisée pour positionner un des points d'extrémité de son parcours de déplacement par rapport au point visé ou de l'axe de visée d'un appareil thérapeutique, de telle sorte que lors de l'irradiation asservie à la respiration, on obtienne une durée proportionnelle d'irradiation de valeur optimale.

17. Procédé selon l'une des revendications 10 à 14, dans lequel la connaissance de la position effective de la cible d'irradiation est utilisée pour effectuer un repositionnement dynamique pendant l'irradiation pour tenir compte de variations de durée moyenne des valeurs aux limites, à savoir du minimum et du maximum du volume des poumons du patient.

18. Procédé selon la revendication 17, dans lequel le déplacement de la cible d'irradiation à l'intérieur du patient est compensé par un déplacement continu et opposé du patient reposant sur un lit.
